(19) Europäisches Patentamt
European Patent Office
Office européen des brevets

(11) Numéro de publication: **0 021 857**
**B1**

(12) **FASCICULE DE BREVET EUROPEEN**

(45) Date de publication du fascicule du brevet: **22.12.82**

(21) Numéro de dépôt: **80400051.1**

(22) Date de dépôt: **15.01.80**

(51) Int. Cl.³: **C 07 D 471/04,**
A 61 K 31/435
//C07D211/86, (C07D471/04, 221/00, 209/00)

(54) **Dérivés de l'indole, leur préparation et medicaments les contenant.**

(30) Priorité: **26.04.79 FR 7910654**

(43) Date de publication de la demande:
**07.01.81 Bulletin 81/1**

(45) Mention de la délivrance du brevet:
**22.12.82 Bulletin 82/51**

(84) Etats contractants désignés:
**AT BE CH DE FR GB IT LU NL SE**

(56) Documents cités:
**EP - A - 0 008 249**
**FR - A - 1 453 532**

**JOURNAL OF ORGANIC CHEMISTRY, vol. 28, no. 11, 12 novembre 1963, pages 3210—12 Columbus, Ohio, U.S.A.**
**G. DE STEVENS et al.: "Investigations in Hetereocycles. XIV. 2- and 3-Azaoctha-hydroindolo(2,3-a)quinolizines"**

(73) Titulaire: **SYNTHELABO**
**58 rue de la Glacière**
**F-75013 Paris (FR)**

(72) Inventeur: **Koletar, Gabor Istvan**
**212 Dogwood Lane**
**Berkeley Heights, New Jersey- 07922 (US)**
Inventeur: **Frost, Jonathan**
**122, Résidence "Les Eaux Vives"**
**F-91120 - Palaiseau (FR)**
Inventeur: **Dupont, Régis**
**141, Bd de la Gare**
**F-75013 Paris (FR)**
Inventeur: **Lardenois, Patrick**
**18, rue Varengue**
**F-92340 - Bourg la Reine (FR)**
Inventeur: **Morel, Claude**
**15, rue de la Bergerie**
**F-91300 - Massy (FR)**
Inventeur: **Najer, Henry**
**2, avenue Emile Acollas**
**F-75007 - Paris (FR)**

(74) Mandataire: **Thouret-Lemaitre, Elisabeth**
**Service Brevets - SYNTHELABO 58, rue de la Glacière**
**F-75621 Paris Cedex 13 (FR)**

**EP 0 021 857 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

Dérivés de l'indole, leur préparation et medicaments les contenant

La présente invention concerne des dérivés de l'indole, leur préparation et leur application en thérapeutique.

La présente invention concerne les compositions pharmaceutiques contenant des pyrido-indoles, sous la forme de racémates ou d'énantiomères optiquement actifs, éventuellement sous la forme de sel d'addition à un acide thérapeutiquement acceptable, répondant à la formule (I)

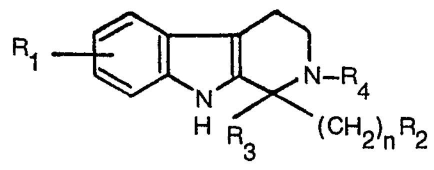

(I)

dans laquelle

n est 0 ou 1,

$R_1$ représente un atome d'hydrogène ou d'halogène, un radical alkyle ou alcoxy ou le groupe $CF_3$,

$R_2$ représente soit un radical alcoxycarbonyle, soit un radical $CONHR_5$, dans lequel $R_5$ est un atome d'hydrogène ou un radical alkyle, cycloalkyle, benzyle, phényle ou méthoxyphényle,

$R_3$ est un atome d'hydrogène, un radical alkyle ou un radical alcoxycarbonyle,

$R_4$ est soit un atome d'hydrogène, soit un radical acyle, soit un radical alkyle, soit un radical $CONHR_6$ dans lequel

$R_6$ est un atome d'hydrogène, un radical alkyle, cycloalkylen benzyle, phényle ou chlorophényle, les alkyles et alcoxy ayant de 1 à 4 atomes de carbone, les cycloalkyles ayant de 3 à 6 atomes de carbone, et les alcoxycarbonyles ayant de 2 à 3 atomes de carbone, à l'exception des compositions contenant un composé de formule (I) dans laquelle, simultanément, n est O, $R_1$ représente un atome d'hydrogène ou un radical alkyle ou alcoxy, $R_2$ représente un radical alcoxycarbonyle, $R_3$ représente un atome d'hydrogène ou un radical alkyle, et $R_4$ représente un atome d'hydrogène ou un radical alkyle ou acyle.

L'invention a également pour objet les composés sous la forme de racémates ou d'isomères optiquement actifs, répondant à la formula (I bis)

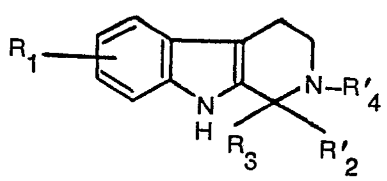

(I bis)

dans laquelle

$R_1$ représente un atome d'hydrogène ou d'halogène, un radical alkyle ou alcoxy ou le groupe $CF_3$,

$R_2$ représente soit un radical $CH_2COO$ alkyle, soit un radical $CONHR_5$, soit un radical $CH_2CONHR_5$, dans lesquels $R_5$ est un atome d'hydrogène ou un radical alkyle, cyloalkyle, benzyle, phényle ou méthoxyphényle,

$R_3$ est un atome d'hydrogène, un radical alkyle ou un radical alcoxycarbonyle,

$R'_4$ est soit un atome d'hydrogène, soit un radical acyle, soit un radical alkyle,

les alkyles et alcoxy ayant de 1 à 4 atomes de carbone, les cycloalkyles ayant de 3 à 6 atomes de carbone, et les alcoxycarbonyles ayant de 2 à 3 atomes de carbone, à l'exception.

a) des composés dans la formule desquels, simultanément,

$R_1$ représente un atome d'hydrogène ou d'halogène ou un radical alkyle ou alcoxy,

$R'_2$ représente un radical méthoxycarbonylméthyle ou éthoxycarbonylméthyle,

$R_3$ représente un atome d'hydrogène ou un radical alkyle,

$R'_4$ représente un atome d'hydrogène, et

b) des composés dans la formule desquels, simultanément,

$R_1$ représente un atome d'hydrogène ou d'halogène ou un radical alkyle ou alcoxy,

$R'_2$ représente un radical $CONHR_5$ ou $CH_2CONHR_5$ où $R_5$ représente un atome d'hydrogène ou un radical alkyle, cycloalkyle ou benzyle,

$R_3$ représente un atome d'hydrogène ou un radical alkyle,

$R'_4$ représente un atome d'hydrogène, et

c) des composés dans la formule desquels, simultanément,

$R_1$ représente un atome d'hydrogène ou d'halogène ou un radical alkyle ou alcoxy,

$R'_2$ représente un radical éthoxycarbonylméthyle,

$R_3$ représente un radical éthoxycarbonyle,

$R'_4$ représente un atome d'hydrogène.

Les composés préférés sont ceux pour lesquels

$R_1$ est H, Cl, F, Br, $CH_3$, $CH_3O$ ou $CF_3$;

$R'_2$ est $CH_2COOCH_3$, $CH_2COOC_2H_5$, $CONHCH_3$, $CH_2CONHCH_3$, $CONHC_2H_5$, ou $CH_2CONHC_2H_5$;

$R_3$ est H, $CH_3$ ou $COOC_2H_5$; et

$R'_4$ est H, $CH_3$ ou $CH_3CO$.

Des composés répondant à la formule (I bis) sont décrits dans J. Org. Chem. *28*, 3210—3212, en tant que composés, ainsi que dans le brevet européen No 8249.

Par ailleurs le brevet français N° 1,453,532 comporte une formule générale couvrant certains composés de l'invention, sans cependant les décrire. Ce même brevet, ainsi que le brevet divisionnaire qui en est issu, N° 5569 M, mentionne que les composés effectivement décrits, et différents de ceux de l'invention, sont utilisables comme substances à activité analgésique.

Les compositions pharmaceutiques et les composés connus sont exclus de l'invention, conformément aux limitations apportées ci-dessus aux définitions concernant les formules (I) et (I bis).

Selon l'invention, on peut préparer les composés de formule (I) conformément aux schémas réactionnels suivants:

Schéma 1 $R_3 \neq H$ $n = 0$ ou 1

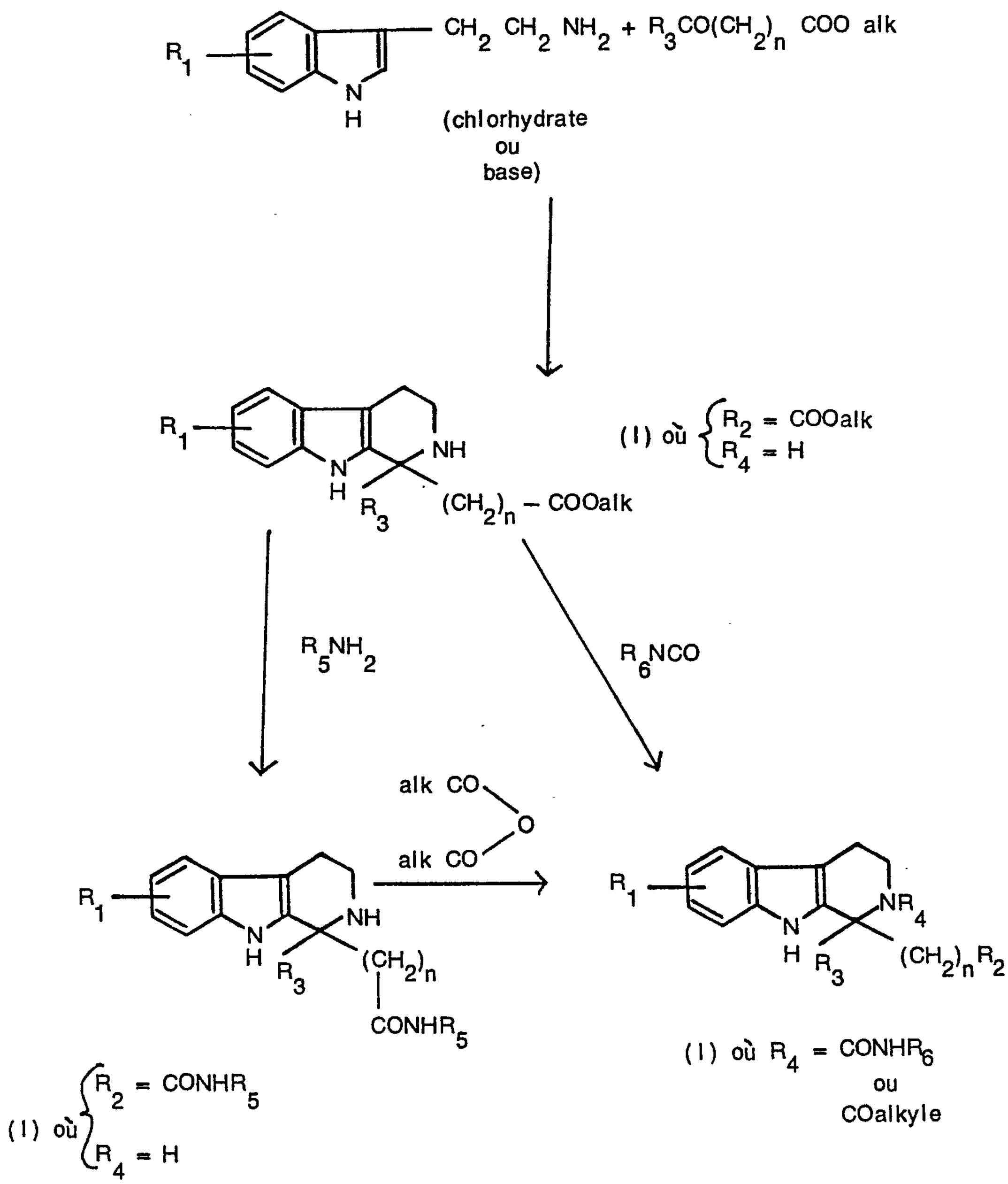

Schéma 2 $R_3$ = H n = 1

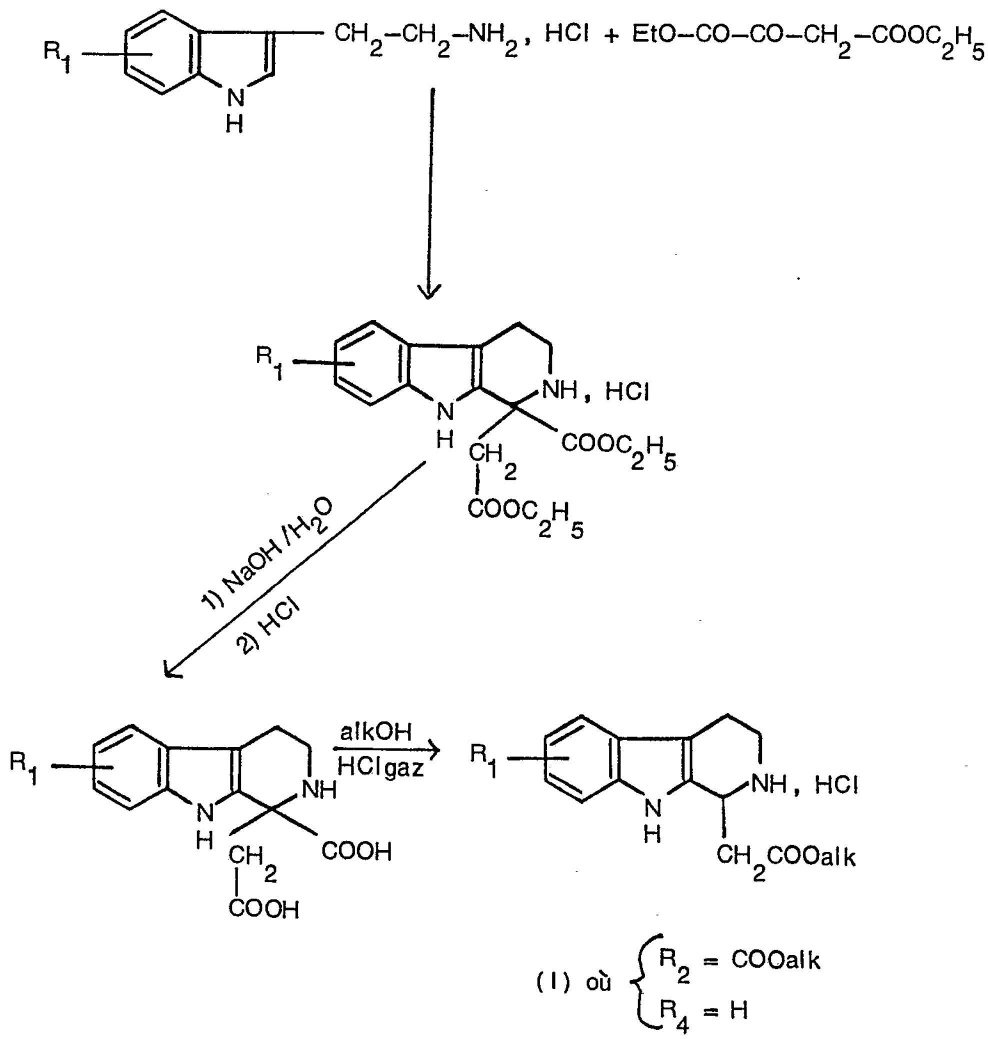


La transformation de l'ester (I) en amide (I) où $R_2 = CONHR_5$ est effectué de la même manière que dans le schéma réactionnel 1; de même l'addition du radical $R_4 = CONHR_6$ sur les composés (I) où $R_2$ = COOalk ou $CONHR_5$ est effectuée de la même manière que dans le schéma 1.

Enfin les composés (I) dans lesquels $R_1$ est $CF_3$ ou Br ont été préparés selon un procédé quelque peu différent (voir exemple 8), adapté à partir du procédé décrit pour $R_1$ = H dans C.A. *60*.5471 h.

Les composés pour lesquels $R_3$ = H et n = 0 sont obtenus selon le mode de préparation décrit par Z. J. Vejdélek et coll., J. of Med. and Pharm. Chem., Vol. 3, N° 3 (1961) p. 427—440.

Les exemples suivants illustrent l'invention. Les microanalyses et les spectres IR et RMN confirment la structure des composés.

Exemple 1

Méthyle-6 tétrahydro-2,3,4,9 1H-pyrido[3,4-b]indole-1-acétate d'éthyle

[n = 1 $R_1 = CH_3$-6 $R_2 = COOC_2H_5$ $R_3$ = H $R_4$ = H]

1. On met en suspension dans 250 ml d'éthanol, 52,52 g (0,25 mole) de chlorhydrate de méthyl-5 tryptamine et on porte à reflux. On met en suspension dans 250 ml d'éthanol, 57,75 g d'éthoxycarbonyl-3 dioxo-1,2 éthoxy-1 propane et on ajoute goutte à goutte en 10 mn, 25 ml d'acide chlorhydrique concentré. On ajoute cette suspension à la suspension de méthyl-5 tryptamine HCl maintenue à la température de reflux. On laisse refroidir pendant la nuit. On élimine le solvant par évaporation et on dissout le résidu dans 400 ml d'eau et on alcalinise avec de l'ammoniaque. Après extraction avec de l'acétate d'éthyle, on obtient une huile que l'on chromatographie sur une colonne de

silice. Après élution avec un mélange 8/2 de chloroforme et d'éthanol, on obtient une huile qui se solidifie par trituration avec de l'éther de pétrole. Après recristallisation dans de l'hexane, le composé obtenu

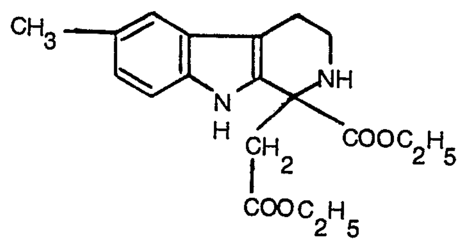

fond à 102—103°C.

2. On chauffe à reflux 45 g du composé précédent dans 450 ml d'une solution aqueuse à 10% de NaOH, pendant 20 h. On ajoute, goutte à goutte, de l'acide chlorhydrique concentré (100 ml) en 30 mn, au mélange réactionnel refroidi. On filtre le solide obtenu et on le sèche sur $P_2O_5$.

3. On chauffe au reflux 99,6 g du solide brut précédemment obtenu dans un mélange de 250 ml d'éthanol et de 20 ml d'acide sulfurique concentré, pendant 9h. On laisse reposer la nuit. On élimine l'éthanol par évaporation et on alcalinise le solide résiduel avec de l'ammoniaque. On extrait avec 3 fois 300 ml d'acétate d'éthyle la solution basique. On évapore. On obtient une huile qui, par trituration avec de l'éther de pétrole, donne un solide blanc. On le filtre et le sèche.

Après recristallisation dans de l'hexane, le composé obtenu fond à 103°C.

### Exemple 2

Chloro-6 méthyl-1 tétrahydro-2,3,4,9 1H-pyrido[3,4-b]indole-1-carboxylate de méthyle

$$[n = 0 \quad R_1 = Cl\text{-}6 \quad R_2 = COOCH_3 \quad R_3 = CH_3 \quad R_4 = H]$$

On fait réagir 36,1 g (0,156 mole) de chlorhydrate de chloro-5 tryptamine en solution dans 350 ml de méthanol avec 20 g de pyruvate de méthyle. On laisse une semaine sous agitation à la température ambiante. On chasse le méthanol au rotavapor le résidu est repris avec de l'acétate d'éthyle. On agite 15 mn puis on filtre le précipité. On traite le précipité avec une solution saturée de bicarbonate de sodium et on extrait à l'acétate d'éthyle. On élimine un insoluble par filtration. On décante la solution organique, on la lave, sèche et évapore au bain-marie sous vide.

Le résidu huileux cristallise après quelques jours. Après recristallisation dans du toluène le composé fond à 148°C.

### Exemple 3

Méthyl-1 méthylaminocarbonyl-2 tétrahydro-2,3,4,9 1H-pyrido (3,4-b]indole-1-acétate d'éthyle

$$[n = 1 \quad R_1 = H \quad R_2 = COOC_2H_5 \quad R_3 = CH_3 \quad R_4 = CONHCH_3]$$

On met en suspension 10,9 g (0,04 mole) de méthyl-1 tétrahydro-2,3,4,9 1H-pyrido[3,4-b]-indole-1-acétate d'éthyle dans 200 ml de cyclohexane. On ajoute 3 ml (0,04 mole) d'isocyanate de méthyle. On chauffe au reflux pendant 1 heure. On laisse refroidir une nuit au réfrigérateur. On filtre le précipité. On recristallise le produit dans de l'éthanol.

F = 217°C.

### Exemple 4

Aminocarbonyl-2 tétrahydro-2,3,4,9 1H-pyrido (3,4-b] indole-1-carboxylate d'éthyle

$$[n = 0 \quad R_1 = H \quad R_2 = COOC_2H_5 \quad R_3 = H \quad R_4 = CONH_2]$$

On chauffe vers 60°C 56,2 g (0,2 mole) de tétrahydro-2,3,4,9 1H-pyrido[3,4-b]indole-1-carboxylate d'éthyle dans 1000 ml d'eau. On ajoute en une seule fois une solution de 15,2 g (0,234 mole) de cyanate de sodium pulvérulent dans 200 ml d'eau. On agite 15 mn puis refroidit vers 10°C. On décante la phase aqueuse réactionnelle, lave le composé avec de l'eau et le recristallise dans de l'éthanol.

F = 215—217°C

Exemple 5

Méthyl-1 méthylaminocarbonyl-1 tétrahydro-2,3,4,9 1H-pyrido[3,4-b]indole

$$[n = 0 \ R_1 = H \ R_2 = CONH \ CH_3 \ R_3 = CH_3 \ R_4 = H]$$

On met 4,8 g (0,02 mole) de méthyl-1 tétrahydro-2,3,4,9, 1H-pyrido[3,4-b]indole-1-carboxylate de méthyle en solution dans 100 ml d'éthanol saturé de méthylamine. On laisse à la température ambiante pendant 48 heures. On élimine le solvant puis reprend le résidu par 20 ml d'éthanol. On filtre et lave à l'éthanol.

F = 230—231 °C.

Exemple 6

Fluoro-6 méthylaminocarbonyl-1 tétrahydro-2,3,4,9 1H-pyrido [3,4-b]indole

$$[n = 1 \ R_1 = F\text{-}6 \ R_2 = CONHCH_3 \ R_3 = H \ R_4 = H]$$

On place dans un autoclave 20 g de fluoro-6 tétrahydro-2,3,4,9 1H-pyrido[3,4-b]indole-1-acétate d'éthyle (obtenu selon le procédé de l'exemple 1) et 500 ml d'éthanol saturé de méthylamine.

On chauffe à 100°C pendant 5h. On chasse le solvant et obtient un solide blanc. On le recristallise dans de l'éthanol.

F = 227°C.

Exemple 7

Chloro-6 méthylaminocarbonyl-1 méthyl-1 acétyl-2 tétrahydro-2,3,4,9 1H-pyrido [3,4-b]indole.

$$[n = 1 \ R_1 = Cl\text{-}6 \ R_2 = CONHCH_3 \ R_3 = CH_3 \ R_4 = COCH_3]$$

On dissout 3 g (0,0102 mole) de chloro-6 méthylaminocarbonyl-1 méthyl-1 tétrahydro-2,3,4,9 1H-pyrido[3,4-b]indole dans 30 ml de pyridine. On ajoute 2 ml d'anhydride acétique. On agite à la température ambiante pendant 48h. On évapore et chasse la pyridine. On reprend le résidu avec 20 ml d'éthanol et on filtre le précipité. Après recristallisation dans l'éthanol le produit fond à 214°C.

Exemple 8

Trifluorométhyl-6 tétrahydro-2,3,4,9 1H-pyrido [3,4-b]indole-1-acétate d'éthyle.

$$[n = 1 \ R_1 = CF_3\text{-}6 \ R_2 = COOC_2H_5 \ R_3 = H \ R_4 = H]$$

Le schéma réactionnel est le suivant:

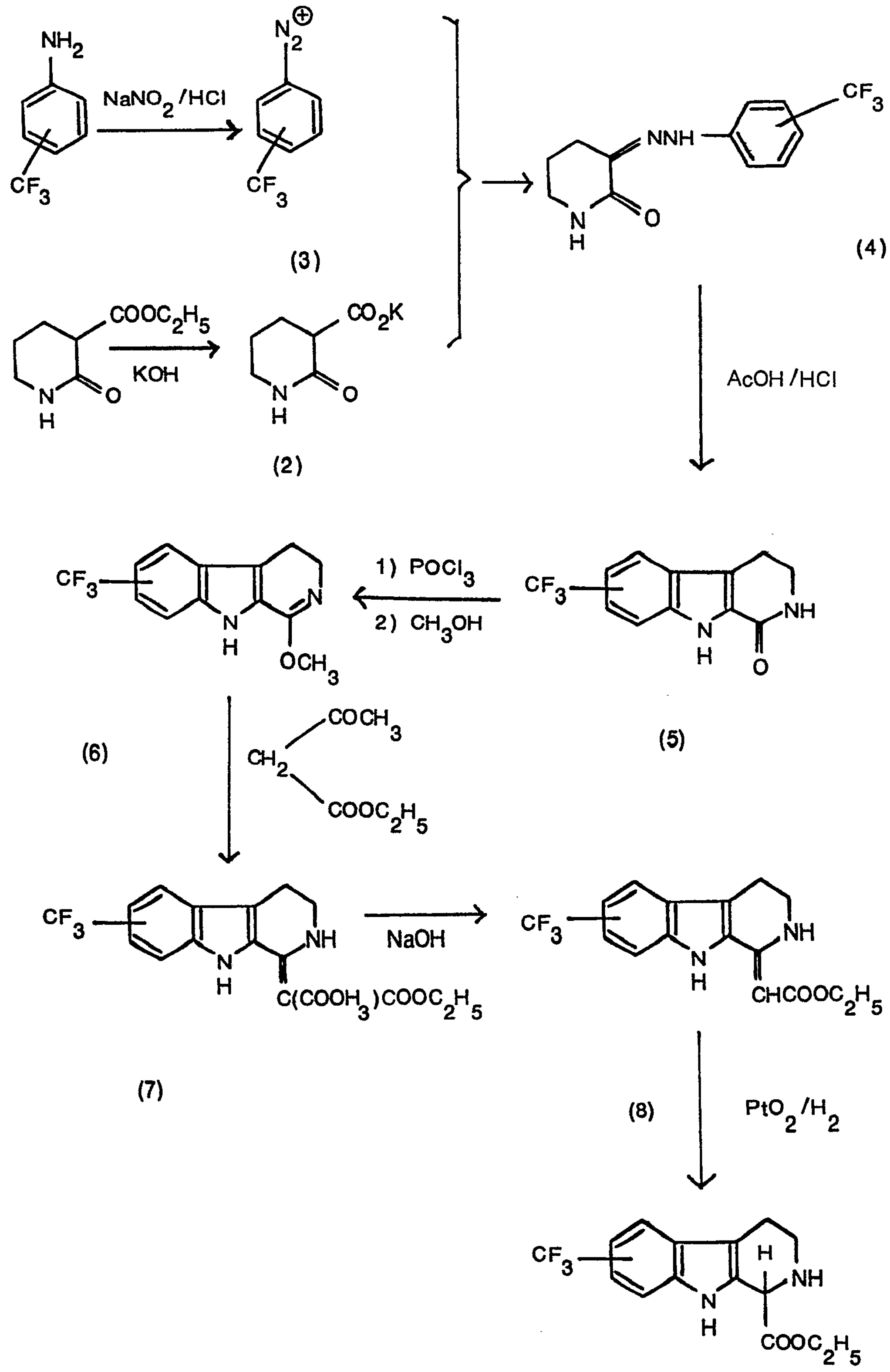


(3) (2) (4) (5) (6) (7) (8) (1)

1) On place dans un ballon 42,75 g (0,25 mole) d'éthoxycarbonyl-3 pyridone-2 et on ajoute 500 ml d'eau. On ajoute 15 g de KOH et one agite à la température ambiante pendant 24h.

2) On fait réagir la trifluorométhyl-4 aniline (42,25 g) en solution dans de l'eau (250 ml) et de l'acide chlorhydrique concentré (55 ml) et avec 18,75 g de $NaNO_2$ dans 125 ml d'eau à une température de 0 à 5°C. Puis on ajoute 50 g de $CH_3COONa$ dans 125 ml d'eau.

3) On ajoute le composé obtenu sous 2 au composé obtenu en 1, en 10 mn, à 0—5°C. On agite pendant 5 mn. On ajoute 75 ml d'acide acétique. On agite 4 h à la température ambiante. On essore et recristallise dans de l'éthanol.

4) On place dans un ballon 20 g du composé

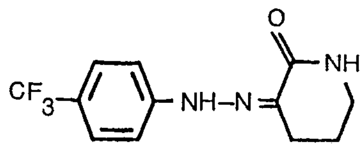

et on ajoute 135 ml d'acide acétique glacial et 68 ml d'acide chlorhydrique. On chauffe à la température de reflux pendant 1h. On refroidit et on verse, le mélange réactionnel sur 300 ml de glace. On recristallise le solide obtenu dans de l'éthanol.

5) On place dans un ballon 15 g de trifluorométhyl-6 oxo-1 tétrahydro-2,3,4,9 1H-pyrido-[3,4-b]-indole et on ajoute 50 ml de $POCl_3$. On agite à la température ambiante pendant 24 h. On ajoute 60 ml d'éther éthylique et on essore. On lave avec 20 ml d'éther éthylique. On sèche le produit sous vide sur $P_2O_5$ pendant 2 h.

On place dans un ballon 60 ml de méthanol et on refroidit à 0°C. On ajoute fraction par fraction 18,3 g du composé obtenu précédemment. On agite à la température ambiante pendant 1 h. On ajoute de l'éther éthylique et on obtient un précipité. Après lavage du composé, on obtient un produit blanc (6) qui fond à 220°C.

6) On place 8,5 g de ce composé dans 1 ballon et on ajoute 25 ml d'acétoacétate d'éthyle. On chauffe sous azote à 140°C pendant 8h. On laisse reposer le mélange réactionnel une nuit. Après lavage, essorage et séchage, on obtient le composé (7) qui fond à 144°C.

7) On place dans un ballon 7,5 g du composé (7) et on ajoute 30 ml d'éthanol. On ajoute une solution de NaOH (1,25 g) dans de l'eau (30 ml). On chauffe à la température de reflux pendant 1 h. On refroidit dans un bain de glace. Après essorage et séchage sous vide suir $P_2O_5$ le composé (8) fond à 192°C.

8) On place dans un autoclave 5,2 g du composé (8) 13 ml d'acide acétique et 50 ml d'éthanol. On ajoute 0,43 g d'oxyde de platine et on agite sous hydrogène sous pression pendant 1 heure et demie. On filtre et on chasse l'éthanol. On alcalinise avec $NH_4OH$ et extrait le solide avec 4 fois 100 ml d'acétate d'éthyle. Après séchage on obtient une huile qui se solidifie. Après recristallisation dans de l'éthanol le composé (I) fond à 140°C.

Les composés de l'invention préparés à titre d'exemples sont représentés dans le tableau suivant.

TABLEAU

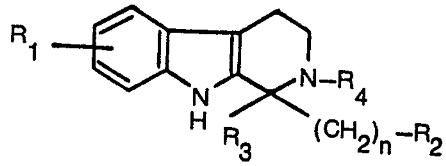

HCl = Chlorhydrate

m.s = Méthane-sulfonate

| Composé | $R_1$ | $R_2$ | $R_3$ | $R_4$ | n | F(°C) |
|---|---|---|---|---|---|---|
| 1 (ex. 1) | $CH_3$–6 | $COOC_2H_5$ | H | H | 1 | 103 |
| 2 | $CH_3$–6 | $COOC_2H_5$ | $COOC_2H_5$ | H | 1 | 102–103 |
| 3 | $CH_3O$–6 | $COOC_2H_5$ | H | H | 1 | 181 (maléate) |
| 4 | $CH_3O$–6 | $COOC_2H_5$ | $COOC_2H_5$ | H | 1 | 100 |
| 5 | H | $COOC_2H_5$ | $COOC_2H_5$ | H | 1 | 80–82 |
| 6 | Cl–6 | $COOC_2H_5$ | $CH_3$ | H | 1 | 104 |
| 7 | Cl–6 | $COOC_2H_5$ | H | H | 1 | 240 m.s |
| 8 | Cl–6 | $COOC_2H_5$ | $COOC_2H_5$ | H | 1 | 101 |
| 9 | F–6 | $COOC_2H_5$ | H | H | 1 | 229–230 m.s |
| 10 | F–6 | $COOC_2H_5$ | $COOC_2H_5$ | H | 1 | 197 HCl |
| 11 (ex.2) | Cl–6 | $COOCH_3$ | $CH_3$ | H | 0 | 148 |
| 12 | H | $COOC_2H_5$ | H | $CONH_2$ | 1 | huile |
| 13 (ex. 3) | H | $COOC_2H_5$ | $CH_3$ | $CONHCH_3$ | 1 | 217 |
| 14 (ex. 4) | H | $COOC_2H_5$ | H | $CONH_2$ | 0 | 215–217 |
| 15 | H | $COOC_2H_5$ | H | $CONHC_2H_5$ | 0 | 190–192 |
| 16 | H | $COOC_2H_5$ | H | $CONHC_3H_7$n | 0 | 170–175 |
| 17 | H | $COOC_2H_5$ | H | $CONHC_4H_9$t | 0 | 209–210 |
| 18 | H | $COOC_2H_5$ | H | $CONHCH_3$ | 0 | 158–162 |
| 19 | H | $COOC_2H_5$ | H | $CONHC_3H_7$i | 0 | 145–150 |
| 20 | H | $COOC_2H_5$ | H | $CONHC_6H_5$ | 0 | 164–168 |
| 21 | H | $COOC_2H_5$ | H | CONH 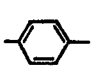 Cl | 0 | 160 |

TABLEAU (Suite)

| Composé | $R_1$ | $R_2$ | $R_3$ | $R_4$ | n | F (°C) |
|---|---|---|---|---|---|---|
| 22 | H | $COOC_2H_5$ | H | $CONHC_4H_9n$ | 0 | 140 |
| 23 | $CF_3$–6 | $COOC_2H_5$ | H | H | 1 | 140 |
| 24 | Br–6 | $COOC_2H_5$ | H | H | 1 | huile |
| 25 | H | $COOC_2H_5$ | $CH_3$ | $-COCH_3$ | 1 | 194 |
| 26 | H | $COOCH_3$ | $CH_3$ | $COCH_3$ | 0 | 238 |
| 27 | H | $COOC_2H_5$ | H | $COCH_3$ | 0 | 211–212 |
| 28 | F–6 | $COOCH_3$ | $CH_3$ | H | 0 | 248 |
| 29 | $CH_3$–6 | $COOCH_3$ | $CH_3$ | H | 0 | 142 |
| 30 | H | $CONHCH_3$ | $CH_3$ | H | 1 | 182 |
| 31 | Cl–6 | $CONHCH_3$ | $CH_3$ | H | 1 | 212 |
| 32 | H | $CONHCH_2$ 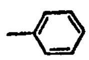 | H | H | 1 | 160 |
| 33 | H | CONH 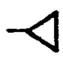 | H | H | 1 | 166–168 |
| 34 (ex. 6) | F–6 | $CONHCH_3$ | H | H | 1 | 227 |
| 35 | Cl–6 | $CONHCH_3$ | H | H | 1 | 232–233 |
| 36 | $CH_3$–6 | $CONHCH_3$ | H | H | 1 | 216 |
| 37 | $CH_3O$–6 | $CONHCH_3$ | H | H | 1 | 190 |
| 38 | H | $CONHC_2H_5$ | H | H | 1 | 148–149 |
| 39 | H | $CONHCH_3$ | $CH_3$ | $CH_3$ | 1 | 240 |
| 40 (ex. 5) | H | $CONHCH_3$ | $CH_3$ | H | 0 | 230 |
| 41 | H | $CONHCH_3$ | $CH_3$ | $CH_3$ | 0 | 207 |
| 42 | Cl–6 | $CONHCH_3$ | $CH_3$ | H | 0 | 230 |
| 43 | F–6 | $CONHCH_3$ | $CH_3$ | H | 0 | 238 |
| 44 (ex. 7) | Cl–6 | $CONHCH_3$ | $CH_3$ | $COCH_3$ | 1 | 214 |
| 45 | Cl–6 | $CONHCH_3$ | $CH_3$ | $COCH_3$ | 0 | 280 |
| 46 | Cl–6 | $CONHC_2H_5$ | H | H | 1 | 240 |
| 47 | Cl–6 | CONH 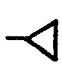 | H | H | 1 | 242 |

TABLEAU (Suite)

| Composé | $R_1$ | $R_2$ | $R_3$ | $R_4$ | n | F (°C) |
|---|---|---|---|---|---|---|
| 48 | Cl–6 | $CONHCH_3$ | $CH_3$ | $COCH_3$ | 1 | 214 |
| 49 | Cl–6 | $CONHCH_3$ | H | $COCH_3$ | 1 | 267 |
| 50 | H | CONH– 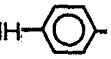 – OCH | H | H | 1 | 189 |
| 51 | H | $CONHC_2H_5$ | $CH_3$ | H | 0 | 159 |
| 52 | H | CONH – 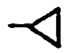 | $CH_3$ | H | 0 | 164 |
| 53 | Cl–6 | $CONHCH_3$ | $CH_3$ | $COCH_3$ | 0 | 280 |
| 54 | $CH_3$–6 | $CONHCH_3$ | $CH_3$ | H | 0 | 214 |
| 55 | H | $CONHCH_3$ | H | H | 0 | 158 |
| 56 | H | $CONH_2$ | H | H | 1 | 198–200 |
| 57 | H | $CONHCH_3$ | H | H | 0 | 158 |

Les composés (I) de l'invention ont été soumis à divers essais pharmacologiques.

Les composés ont en effet été soumis au test de l'anoxie hypobare chez la souris et au test de l'antagonisme vis-à-vis de la ptose réserpinique (Gouret C. et al., J. Pharmacol. (Paris) *8*, 333—350 (1977).

ANOXIE HYPOBARE

Des souris de souche CDI sont maintenues dans une atmosphère appauvrie en oxygène, par réalisation d'un vide partiel (190 mm de mercure correspondant à 5,25% d'oxygène).

Le temps de survie des animaux est noté. Ce temps est augmenté par les agents capables de favoriser l'oxygénation tissulaire et en particulier cérébrale. Les composés étudiés sont administrés à plusieurs doses, par voie intrapéritonéale, 10 minutes avant l'essai. Les pourcentages d'augmentation du temps de survie par rapport aux valeurs obtenues chez les animaux témoins sont calculés. La dose active moyenne (DAM), dose qui augmente le temps de survie de 100% est déterminée graphiquement.

La DAM des composés de l'invention varie de 13 à 26 mg/kg par voie i.p.

ACTIVITE ANTIDEPRESSIVE

L'activité antidépressive a été déterminée par le test de l'antagonisme vis-à-vis de la ptose réserpinique (Gouret C. et al., J. Pharmacol. (Paris) *8*, 333—350 (1977).

Les souris (mâles, CDI Charles River, France, 18—22 g) reçoivent simultanément les produits à étudier ou le solvant (*voie i.p.*), et la réserpine (4 mg/kg, *voie s.c.)*.

Soixante minutes plus tard, le degré de ptose palpébrale est estimé au moyen d'une échelle de cotation (0 à 4), pour chaque souris.

A chaque dose, la moyenne de cotation et le pourcentage de variation par rapport au lot contrôle, sont calculés.

Pour chaque produit, la $DA_{50}$, ou dose qui diminue de 50% le score moyen de ptose par rapport aux contrôles, est déterminée graphiquement.

La $DA_{50}$ varie de 2 à 10 mg/kg par voie i.p.

ACTION SUR LA DUREE DU "SOMMEIL"

Cette action a été déterminée par l'influence des composés sur la durée du "sommeil" induit par le 4-hydroxy-butyrate de sodium (GHB) chez le rat curarisé sous respiration artificielle dans lequel

l'activité électrocorticographique est enregistrée par des électrodes corticales.

Les composés de l'invention diminuent la durée totale du sommeil de 20 à 35%.

L'étude pharmacologique des composés de l'invention montre qu'ils sont actifs dans l'épreuve d'anoxie hypobare chez la souris tout en n'étant que peu toxiques et qu'ils exercent une action, significative éveillante dans le test du "sommeil" induit par le 4-hydroxy-butyrate de sodium.

Les composés de l'invention, possédant à la fois une activité antianoxique et une activité psychotrope, peuvent être utilisés en thérapeutique pour le traitement des troubles de la vigilance, en particulier pour lutter contre les troubles du comportement imputables à des dommages vasculaires cérébraux et à la sclérose cérébrale en gériatrie, ainsi que pour le traitement des absences dues à des traumatismes crâniens, et le traitement des états dépressifs.

L'invention comprend par conséquent, toutes compositions pharmaceutiques, renfermant les composés et/ou leurs sels comme principes actifs, en association avec tous excipients appropriés à leur administration, en particulier par voie orale ou parentérale.

Les voies d'administration peuvent être les voies orale et parentérale.

La posologie quotidienne peut aller de 10 à 1000 mg.

**Revendications pour les Etats contractants: BE CH DE FR GB IT LU NL SE**

1. Compositions pharmaceutiques utilisables dans le traitement des troubles de la vigilance et des états dépressifs, caractérisées en ce qu'elles contiennent les pyrido-indoles, sous la forme de racémates ou d'isomères optiquement actifs, éventuellement sous la forme d'un sel d'addition à un acide pharmaceutiquement acceptable, répondant à la formule (I)

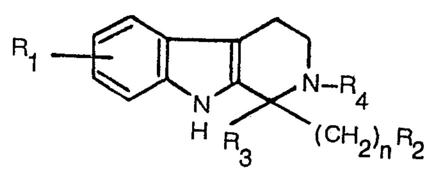

dans laquelle

n est 0 ou 1,

$R_1$ représente un atome d'hydrogène ou d'halogène, un radical alkyle ou alcoxy ou le groupe $CF_3$,

$R_2$ représente soit un radical alcoxycarbonyle, soit un radical $CONHR_5$, dans lequel $R_5$ est un atome d'hydrogène ou un radical alkyle, cycloalkyle, benzyle, phényle ou méthoxyphényle,

$R_3$ représente un atome d'hydrogène, un radical alkyle ou alcoxycarbonyle,

$R_4$ représente un atome d'hydrogène, un radical alkyle, un radical acyle ou un radical $CONHR_6$ dans lequel $R_6$ est un atome d'hydrogène ou un radical alkyle, cycloalkyle, benzyle, phényle ou chlorophényle,

les alkyles, acyles et alcoxy ayant de 1 à 4 atomes de carbone, les alcoxycarbonyles ayant de 2 à 3 atomes de carbone, et les cycloalkyles ayant de 3 à 6 atomes de carbone,

à l'exception des compositions contenant un composé de formule (I) dans laquelle, simultanément, n est 0,

$R_1$ représente un atome d'hydrogène ou un radical alkyle ou alcoxy, $R_2$ représente un radical alkoxycarbonyle, $R_3$ représente un atome d'hydrogène ou un radical alkyle, et $R_4$ représente un atome d'hydrogène ou un radical alkyle ou acyle.

2. Pyrido-indoles, sous la forme de racémates ou d'isomères optiquement actifs, répondant à la formule (I bis)

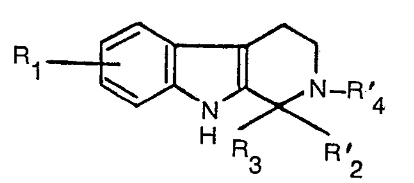

(I bis)

dans laquelle

$R_1$ représente un atome d'hydrogène ou d'halogène, un radical alkyle ou alcoxy ou le groupe $CF_3$,

$R'_2$ représente soit un radical $CH_2COO$ alkyle, soit un radical $CONHR_5$, soit un radical $CH_2CONHR_5$, dans lesquels $R_5$ est un atome d'hydrogène ou un radical alkyle, cyloalkyle, benzyle, phényle ou méthoxyphényle,

$R_3$ représente un atome d'hydrogène, un radical alkyle ou un radical alcoxycarbonyle,

R'$_4$ représente un atome d'hydrogène, un radical alkyle ou un radical acyle, les alkyles, acyles et alcoxy ayant de 1 à 4 atomes de carbone, les alkoxycarbonyles ayant de 2 à 3 atomes de carbone, et les cycloalkyles ayant de 3 à 6 atomes de carbone, à l'exception

a) des composés dans la formule desquels, simultanément,

$R_1$ représente un atome d'hydrogène ou d'halogène ou un radical alkyle ou alcoxy,

R'$_2$ représente un radical méthoxycarbonylméthyle ou éthoxycarbonylméthyle,

$R_3$ représente un atome d'hydrogène ou un radical alkyle,

R'$_4$ représente un atome d'hydrogène, et

b) des composés dans la formule desquels, simultanément,

$R_1$ représente un atome d'hydrogène ou d'halogène ou un radical alkyle ou alcoxy,

R'$_2$ représente un radical $CONHR_5$ ou $CH_2CONHR_5$ où $R_5$ représente un atome d'hydrogène ou un radical alkyle, cycloalkyle ou benzyle,

$R_3$ représente un atome d'hydrogène ou un radical alkyle,

R'$_4$ représente un atome d'hydrogène, et

c) des composés dans la formule desquels, simultanément,

$R_1$ représente un atome d'hydrogène ou d'halogène ou un radical alkyle ou alcoxy,

R'$_2$ représente un radical éthoxycarbonylméthyle,

$R_3$ représente un radical éthoxycarbonyle,

R'$_4$ représente un atome d'hydrogène.

3. Pyridoindoles selon la revendication 2, dans la formule desquels

$R_1$ est H, Cl, F, Br, $CH_3$, $CH_3O$ ou $CF_3$;

R'$_2$ est $CH_2COOCH_3$, $CH_2COOC_2H_5$, $CONHCH_3$, $CH_2CONHCH_3$, $CONHC_2H_5$, ou $CH_2CONHC_2H_5$;

$R_3$ est H, $CH_3$ ou $COOC_2H_5$; et

R'$_4$ est H, $CH_3$ ou $CH_3CO$.

4. Procédé de préparation des composés définis dans la revendication 2, pour lesquels $R_3$ est différent de H, caractérisé en ce qu'on fait réagir un composé de formule

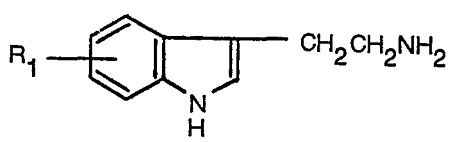

avec un composé de formule $R_3CO(CH_2)_nCOOalk$, n étant 0 ou 1 et alk un radical alkyle, puis, si on le désire, on fait réagir le composé obtenu de formule

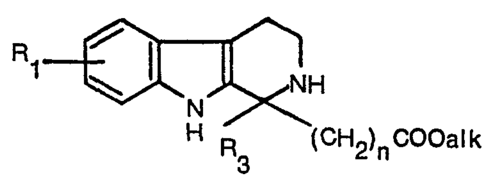

soit avec une amine $R_5NH_2$ pour obtenir un composé dans la formule (I bis) duquel R'$_2$ représente $CONHR_5$ ou $CH_2CONHR_5$, et/ou avec un composé R'$_4$X (X = anion) pour obtenir un composé dans la formule (I bis) duquel R'$_4$ représente un radical alkyle ou acyle, les symboles $R_1$, R'$_2$, $R_3$, R'$_4$, et $R_5$ étant définis comme dans la revendication 2.

5. Procédé de préparation des composés définis dans la revendication 2, pour lesquels R'$_2$ représente $CH_2COO$ alkyle ou $CH_2CONHR_5$ et $R_3$ représente H, caractérisé en ce qu'on fait réagir un composé de formule

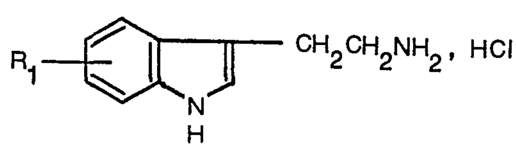

avec le composé $C_2H_5O—CO—CO—CH_2—CO—OC_2H_5$ pour obtenir un composé de formule

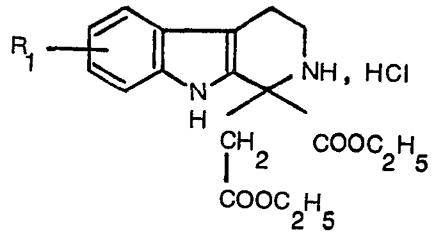

que l'on saponifie en diacide de formule

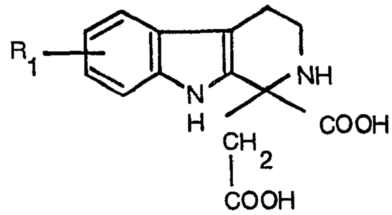

que l'on fait réagir avec un alcool alkOH pour obtenir un composé de formule I bis,

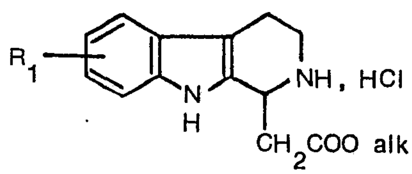

alk étant un radical alkyle, et, si on le désire, on fait réagir ce composé (I bis) soit avec une amine $R_5NH_2$ pour obtenir un composée (I bis) duquel $R'_2$ représente $CH_2CONHR_5$, et/ou avec un composé $R'_4$ X (X = anion) pour obtenir un composé dans la formule (I bis) duquel $R'_4$ représente un radical alkyle ou acyle les symboles, $R_1$, $R'_4$ et $R_5$ étant définis comme dans la revendication 2.

**Revendications pour l'Etat contractant: AT**

1. Procédé de préparation de pyrido-indoles sous la forme de racémates ou d'isomères optiquement actifs, répondant à la formule (I bis)

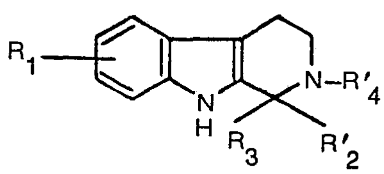

(I bis)

dans laquelle

$R_1$ représente un atome d'hydrogène ou d'halogène, un radical alkyle ou alcoxy ou le groupe $CF_3$,

$R'_2$ représente soit un radical $CH_2COO$ alkyle, soit un radical $CONHR_5$, soit un radical $CH_2CONHR_5$, dans lesquels $R_5$ est un atome d'hydrogène ou un radical alkyle, cyloalkyle, benzyle, phényle ou méthoxyphényle,

$R_3$ représente un radical alkyle ou un radical alcoxycarbonyle,

$R'_4$ représente un atome d'hydrogène, un radical alkyle ou un radical acyle,

les alkyles, acyles et alcoxy ayant de 1 à 4 atomes de carbone, les alkoxycarbonyles ayant de 2 à 3 atomes de carbone, et les cycloalkyles ayant de 3 à 6 atomes de carbone, à l'exception

a) des composés dans la formule desquels, simultanément,

$R_1$ représente un atome d'hydrogène ou d'halogène ou un radical alkyle ou alcoxy,

$R'_2$ représente un radical méthoxycarbonylméthyle ou éthoxycarbonylméthyle,

$R_3$ représente un radical alkyle,

$R'_4$ représente un atome d'hydrogène, et

b) des composés dans la formule desquels, simultanément,

$R_1$ représente un atome d'hydrogène ou d'halogène ou un radical alkyle ou alcoxy,

$R'_2$ représente un radical $CONHR_5$ ou $CH_2CONHR_5$ où $R_5$ représente un atome d'hydrogène ou un radical alkyle, cycloalkyle ou benzyle,

$R_3$ représente un radical alkyle,

$R'_4$ représente un atome d'hydrogène, et

c) des composés dans la formule desquels, simultanément,

$R_1$ représente un atome d'hydrogène ou d'halogène ou un radical alkyle ou alcoxy,

$R'_2$ représente un radical éthoxycarbonylméthyle,

$R_3$ représente un radical éthoxycarbonyle,

$R'_4$ représente un atome d'hydrogène, procédé caractérisé en ce qu'on fait réagir un composé de formule

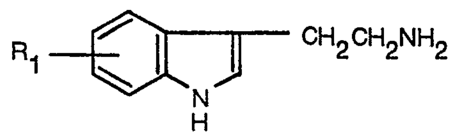

avec un composé de formule $R_3CO(CH_2)_nCOOalk$, n étant 0 ou 1 et alk un radical alkyle, puis, si on le désire, on fait réagir le composé obtenu de formule

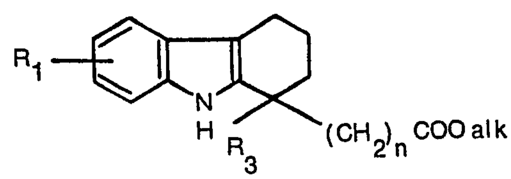

soit avec une amine $R_5NH_2$ pour obtenir un composé dans la formule (I bis) duquel $R'_2$ représente $CONHR_5$ ou $CH_2CONHR_5$, et/ou avec un composé $R'_4$ X (X = anion) pour obtenir un composé dans la formule (I bis) duquel $R'_4$ représente un radical alkyle ou acyle, les symboles $R_1$, $R'_2$, $R_3$, $R'_4$, et $R_5$ étant définis comme ci-dessus

2. Procédé de préparation de pyrido-indoles sous la forme de racémates ou d'isomères optiquement actifs, répondant à la formule (I bis)

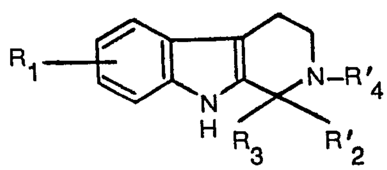

dans laquelle

$R_1$ représente un atome d'hydrogène ou d'halogène, un radical alkyle ou alcoxy ou le groupe $CF_3$,

$R'_2$ représente soit un radical $CH_2COO$ alkyle soit un radical $CH_2CONHR_5$, dans lequel $R_5$ est un atome d'hydrogène ou un radical alkyle, cycloalkyle, benzyle, phényle ou méthoxyphényle,

$R_3$ représente un atome d'hydrogène,

$R'_4$ représente un atome d'hydrogène, un radical alkyle ou un radical acyle,

les alkyles, acyles et alcoxy ayant de 1 à 4 atomes de carbone, les alkoxycarbonyles ayant de 2 à 3 atomes de carbone, et les cycloalkyles ayant de 3 à 6 atomes de carbone, à l'exception

a) des composés dans la formule desquels, simultanément,

$R_1$ représente un atome d'hydrogène ou d'halogène ou un radical alkyle ou alcoxy,

$R'_2$ représente un radical méthoxycarbonylméthyle ou éthoxycarbonylméthyle,

$R'_4$ représente un atome d'hydrogène, et

b) des composés dans la formule desquels, simultanément,

$R_1$ représente un atome d'hydrogène ou d'halogène ou un radical alkyle ou alcoxy,

$R'_2$ représente un radical $CONHR_5$ ou $CH_2CONHR_5$ où $R_5$ représente un atome d'hydrogène ou un radical alkyle, cycloalkyle ou benzyle et

$R'_4$ représente un atome d'hydrogène, procédé caractérisé en ce qu'on fait réagir un composé de formule

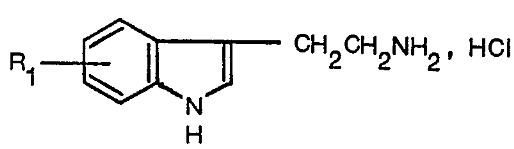

avec le composé $C_2H_5O—CO—CO—CH_2—CO—OC_2H_5$ pour obtenir un composé de formule

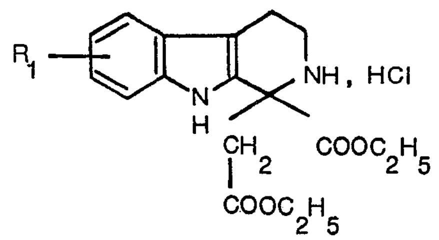

que l'on saponifie en diacide de formule

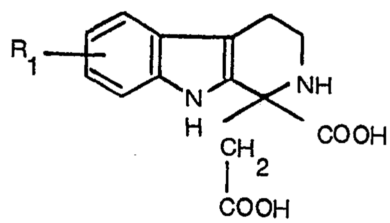

que l'on fait réagir avec un alcool alkOH pour obtenir un composé de formule I bis,

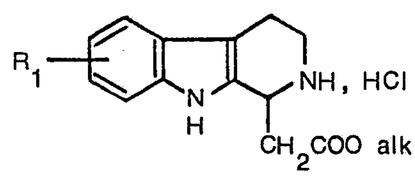

alk étant un radical alkyle, et, si on le désire, on fait réagir ce composé (I bis) soit avec un amine $R_5NH_2$ pour obtenir un composé (I bis) duquel $R'_2$ représente $CH_2CONHR_5$, et/ou avec un composé $R'_4$ X (X = anion) pour obtenir un composé dans la formule (I bis) duquel $R'_4$ représente un radical alkyle ou acyle, les symboles, $R_1$, $R'_4$ et $R_5$ étant définis comme ci-dessus

**Claims for the Contracting States: BE CH DE FR GB IT LU NL SE**

1. Pharmaceutical compositions suitable for treating vigilance disorders and depressive states, characterized in that they contain the pyrido-indoles, in the form of racemates or optically active isomers, optionally in the form of a pharmaceutically acceptable acid addition salt, corresponding to formula (I)

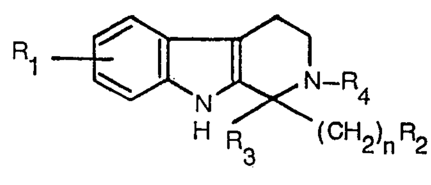

wherein

n is 0 or 1,

$R_1$ is a hydrogen or halogen atom, an alkyl or alkoxy radical or the $CF_3$-group,

$R_2$ is either an alkoxycarbonyl radical or a $CONHR_5$ radical wherein $R_5$ is a hydrogen atom or an alkyl, cycloalkyl, benzyl, phenyl or methoxyphenyl radical,

$R_3$ is a hydrogen atom, an alkyl or alkoxycarbonyl radical,

$R_4$ is a hydrogen atom, an alkyl radical, an acyl radical or a $CONHR_6$ radical wherein $R_6$ is a hydrogen atom or an alkyl cycloalkyl, benzyl, phenyl or chlorophenyl radical,

said alkyls, acyls and alkoxy having 1 to 4 carbon atoms, said alkoxycarbonyls having 2 to 3 carbon

atoms, and said cycloalkyls having 3 to 6 carbon atoms, with the exception of the compositions containing a compound of formula (I) wherein, simultaneously, n is 0, $R_1$ is a hydrogen atom or an alkyl or alkoxy radical, $R_2$ is an alkoxycarbonyl radical, $R_3$ is a hydrogen atom or an alkyl radical, and $R_4$ is a hydrogen atom or an alkoxy or acyl radical.

2. Pyrido-indoles, in the form of racemates or optically active isomers, corresponding to formula (I bis)

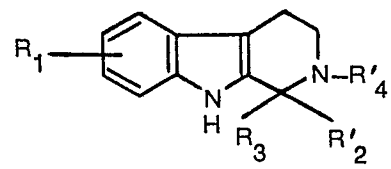

(I bis)

wherein

$R_1$ is a hydrogen or halogen atom, an alkyl or alkoxy radical, or the $CF_3$-group,

$R'_2$ is either a $CH_2COO$alkyl radical, or a $CONHR_5$ radical, or a $CH_2CONHR_5$ radical, wherein $R_5$ is a hydrogen atom or an alkyl, cycloalkyl, benzyl, phenyl or methoxyphenyl radical,

$R_3$ is a hydrogen atom, an alkyl radical, or an alkoxycarbonyl radical,

$R'_4$ is a hydrogen atom, an alkyl radical or an acyl radical,

said alkyls, acyls and alkoxy having 1 to 4 carbon atoms, said alkoxycarbonyls having 2 to 3 carbon atoms, and said cycloalkyls having 3 to 6 carbon atoms, with the exception of

a) the compounds in the formula of which, simultaneously,

$R_1$ is a hydrogen or halogen atom or an alkyl or alkoxy radical,

$R'_2$ is a methoxycarbonylmethyl or ethoxycarbonylmethyl radical,

$R_3$ is a hydrogen atom or an alkyl radical,

$R'_4$ is a hydrogen atom, and

b) the compounds in the formula of which, simultaneously,

$R_1$ is a hydrogen or halogen atom or an alkyl or alkoxy radical,

$R'_2$ is a $CONHR_5$ or $CH_2CONHR_5$ radical wherein $R_5$ is a hydrogen atom or an alkyl, cycloalkyl or benzyl radical,

$R_3$ is a hydrogen atom or an alkyl radical,

$R'_4$ is a hydrogen atom, and

c) the compounds in the formula of which, simultaneously,

$R_1$ is a hydrogen or halogen atom or an alkyl or alkoxy radical,

$R'_2$ is an ethoxycarbonylmethyl radical,

$R_3$ is an ethoxycarbonyl radical,

$R'_4$ is a hydrogen atom.

3. Pyrido-indoles according to claim 2, in the formula of which

$R_1$ is H, Cl, F, Br, $CH_3$, $CH_3O$ or $CF_3$;

$R'_2$ is $CH_2COOCH_3$, $CH_2COOC_2H_5$, $CONHCH_3$, $CH_2CONHCH_3$, $CONHC_2H_5$, or $CH_2CONHC_2H_5$;

$R_3$ is H, $CH_3$ or $COOC_2H_5$; and

$R'_4$ is H, $CH_3$ or $CH_3CO$.

4. A process for preparing the compounds specified in claim 2, wherein $R_3$ is different from H, characterized in that a compound of formula

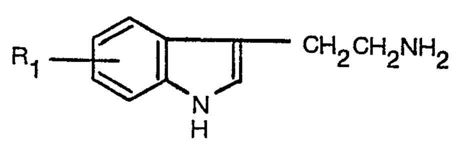

is reacted with a compound of formula $R_3CO(CH_2)_nCOO$alk, n being 0 or 1 and alk being an alkyl radical, then, if desired, the compound thus obtained, of formula

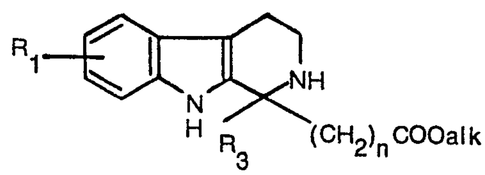

is reacted either with an amine $R_5NH_2$ so as to produce a compound in the formula (I bis) of which $R'_2$ is $CONHR_5$ or $CH_2CONHR_5$, and/or with a compound $R'_4X$ (X = anion) so as to produce a compound in the

formula (I bis) of which $R'_4$ is an alkyl or acyl radical, the symbols $R_1$, $R'_2$, $R_3$, $R'_4$ and $R_5$ being as specified in claim 2.

5. A process for preparing compounds specified in claim 2, wherein $R'_2$ is $CH_2COO$alkyl or $CH_2CONHR_5$ and $R_3$ is H, characterized in that a compound of formula

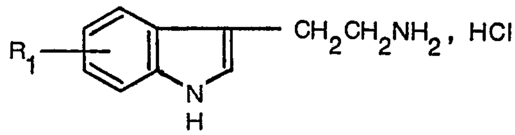

is reacted with the compound $C_2H_5O—CO—CO—CH_2—CO—OC_2H_5$ so as to produce a compound of formula

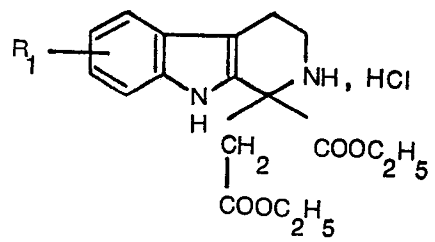

which is saponified into a diacid of formula

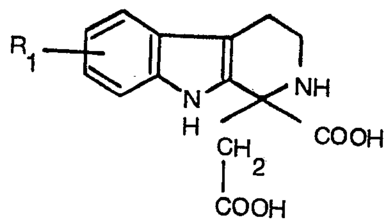

which is reacted with an alcohol alkOH so as to produce a compound of formula I bis

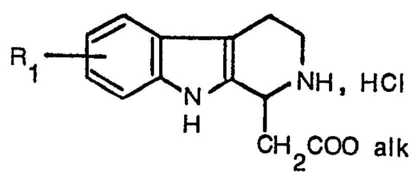

alk being an alkyl radical, and, if desired, said compound (I bis) is reacted either with an amine $R_5NH_2$ so as to produce a compound in the formula (I bis) of which $R'_2$ is $CH_2CONHR_5$, and/or with a compound $R'_4$ X (X = anion) so as to produce a compound in the formula (I bis) of which $R'_4$ is an alkyl or acyl radical, the symbols $R_1$, $R'_4$ and $R_5$ being as specified in claim 2.

**Claims for the Contracting State: AT**

1. A process for preparing pyrido-indoles, in the form of racemates or optically active isomers, corresponding to formula (I bis)

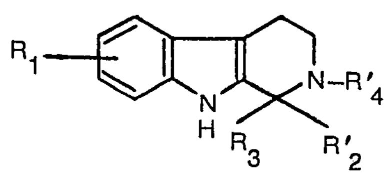

(I bis)

wherein

$R_1$ is a hydrogen or halogen atom, an alkyl or alkoxy radical, or the $CF_3$-group,

$R'_2$ is either a $CH_2COO$alkyl radical, or a $CONHR_5$ radical, or a $CH_2CONHR_5$ radical, wherein $R_5$ is a hydrogen atom or an alkyl, cycloalkyl, benzyl, phenyl or methoxyphenyl radical,

$R_3$ is an alkyl radical, or an alkoxycarbonyl radical,

$R'_4$ is a hydrogen atom, an alkyl radical or an acyl radical,

said alkyls, acyls and alkoxy having 1 to 4 carbon atoms, said alkoxycarbonyls having 2 to 3 carbon atoms, and said cycloalkyls having 3 to 6 carbon atoms, with the exception of

a) the compounds in the formula of which, simultaneously,

$R_1$ is a hydrogen or halogen atom or an alkyl or alkoxy radical,

$R'_2$ is a methoxycarbonylmethyl or ethoxycarbonylmethyl radical,

$R_3$ is an alkyl radical,

$R'_4$ is a hydrogen atom, and

b) the compounds in the formula of which, simultaneously,

$R_1$ is a hydrogen or halogen atom or an alkyl or alkoxy radical,

$R'_2$ is a $CONHR_5$ or $CH_2CONHR_5$ radical wherein $R_5$ is a hydrogen atom or an alkyl, cycloalkyl or benzyl radical,

$R_3$ is an alkyl radical,

$R'_4$ is a hydrogen atom, and

c) the compounds in the formula of which, simultaneously,

$R_1$ is a hydrogen or halogen atom or an alkyl or alkoxy radical,

$R'_2$ is an ethoxycarbonylmethyl radical,

$R_3$ is an ethoxycarbonyl radical,

$R'_4$ is a hydrogen atom, process characterized in that a compound of formula

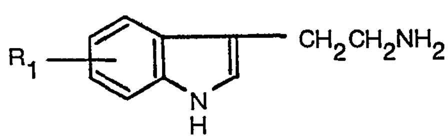

is reacted with a compound of formula $R_3CO(CH_2)_nCOO$alk, n being 0 or 1 and alk being an alkyl radical, then, if desired, the compound thus obtained, of formula

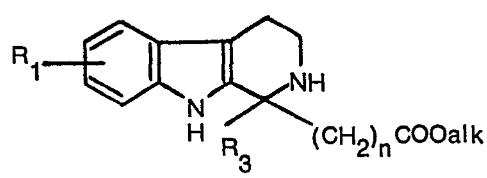

is reacted either with an amine $R_5NH_2$ so as to produce a compound in the formula (I bis) of which $R'_2$ is $CONHR_5$ or $CH_2CONHR_5$, and/or with a compound $R'_4X$ (X = anion) so as to produce a compound in the formula (I bis) of which $R'_4$ is an alkyl or acyl radical, the symbols $R_1$, $R'_2$, $R_3$, $R'_4$ and $R_5$ being as specified above.

2. A process for preparing pyrido-indoles, in the form of racemates or optically active isomers, corresponding to formula (I bis)

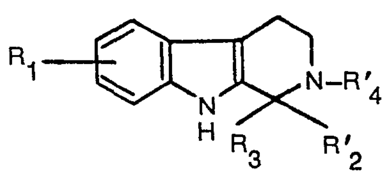

wherein

$R_1$ is a hydrogen or halogen atom, an alkyl or alkoxy radical, or the $CF_3$-group,

$R'_2$ is either a $CH_2COO$alkyl radical or a $CH_2CONHR_5$ radical wherein $R_5$ is a hydrogen atom or an alkyl, cycloalkyl, benzyl, phenyl or methoxyphenyl radical,

$R_3$ is a hydrogen atom,

$R'_4$ is a hydrogen atom, an alkyl radical or an acyl radical, said alkyls, acyls and alkoxy having 1 to 4 carbon atoms, said alkoxycarbonyls having 2 to 3 carbon atoms, and said cycloalkyls having 3 to 6 carbon atoms, with the exception of

a) the compounds in the formula of which, simultaneously,

$R_1$ is a hydrogen or halogen atom or an alkyl or alkoxy radical,
$R'_2$ is a methoxycarbonylmethyl or ethoxycarbonylmethyl radical,
$R'_4$ is a hydrogen atom, and

b) the compounds in the formula of which, simultaneously,
$R_1$ is a hydrogen or halogen atom or an alkyl or alkoxy radical,
$R'_2$ is a $CONHR_5$ or $CH_2CONHR_5$ radical wherein $R_5$ is a hydrogen atom or an alkyl, cycloalkyl or benzyl radical, and
$R'_4$ is a hydrogen atom, process characterized in that a compound of formula

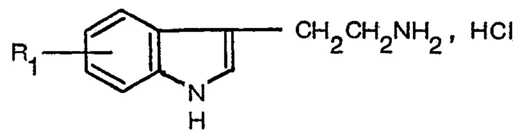

is reacted with the compound $C_2H_5O—CO—CO—CH_2—CO—OC_2H_5$ so as to produce a compound of formula

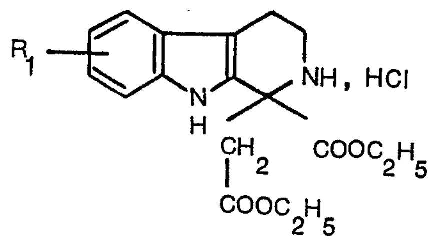

which is saponified into a diacid of formula

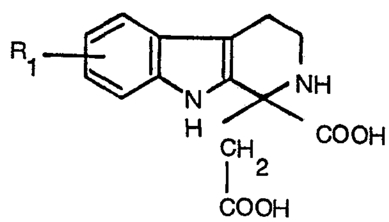

which is reacted with an alcohol alkOH so as to produce a compound of formula I bis

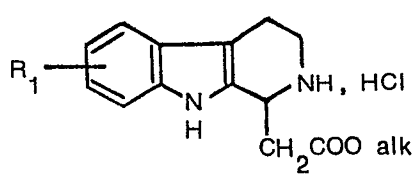

alk being an alkyl radical, and, if desired, said compound (I bis) is reacted either with an amine $R_5NH_2$ so as to produce a compound in the formula (I bis) of which $R'_2$ is $CH_2CONHR_5$, and/or with a compound $R'_4$ X (X = anion) so as to produce a compound in the formula (I bis) of which $R'_4$ is an alkyl or acyl radical, the symbols $R_1$, $R'_4$ and $R_5$ being as specified above.

**Patentansprüche für die Vertragsstaaten: BE CH DE FR GB IT LU NL SE**

1. Pharmazeutische Zusammensetzungen, die zur Behandlung von Wachsamkeitsstörungen und depressiven Zuständen geeignet sind, dadurch gekennzeichnet, daß sie Pyrido-Indole in Form ihrer Racemate oder optisch aktiven Isomeren, gegebenenfalls in Form ihrer Säureadditionssalze mit einer pharmazeutisch anwendbaren Säure enthalten, die der allgemeinen Formel I

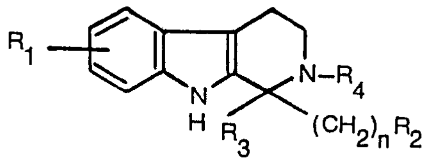

entsprechen, in der n für 0 oder 1,

$R_1$ für Wasserstoff, Halogen, einen Alkyl- oder Alkoxyrest oder die Trifluormethylgruppe,

$R_2$ für einen Alkoxycarbonylrest oder den Rest $CONHR_5$, in dem $R_5$ Wasserstoff oder einen Alkyl-, Cycloalkyl-, Benzyl-, Phenyl- oder Methoxyphenylrest bedeutet,

$R_3$ für Wasserstoff oder einen Alkyl- oder Alkoxycarbonylrest und

$R_4$ für Wasserstoff, einen Alkyl- oder Acylrest oder die Gruppe $CONHR_6$ steht, in welcher $R_6$ Wasserstoff oder einen Alkyl-, Cycloalkyl, Benzyl-, Phenyl- oder Chlorphenylrest bedeutet,

wobei die Alkyl-, Acyl- und Alkoxygruppen 1 bis 4 C-Atome, die Alkoxycarbonylgruppen 2 bis 3 C-atome und die Cycloalkylgruppen 3 bis 6 C-Atome aufweisen, mit der Ausnahme von Zusammensetzungen, die eine Verbindung der Formel I enthalten, in der gleichzeitig n für 0, $R_1$ für Wasserstoff oder eine Alkyl- oder Alkoxygruppe, $R_2$ für einen Alkoxycarbonylrest, $R_3$ für Wasserstoff oder einen Alkylrest und $R_4$ für Wasserstoff oder einen Alkyl- oder Acylrest steht.

2. Pyrido-Indole in Form ihrer Racemate oder optisch aktiven Isomeren, die der allgemeinen Formel (I bis)

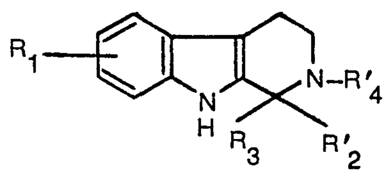

(I bis)

entsprechen, in welcher $R_1$ für Wasserstoff, Halogen, einen Alkyl-, Alkoxy- oder den Trifluoromethylrest,

$R'_2$ für eine $CH_2COO$-Alkylgruppe oder die Reste $CONHR_5$ oder $CH_2CONHR_5$, in welchen $R_5$ Wasserstoff, einen Alkyl-, Cycloalkyl-, Benzyl-, Phenyl- oder Methoxyphenylrest bedeutet,

$R_3$ für Wasserstoff oder einen Alkyl- oder Alkoxycarbonylrest und

$R'_4$ für Wasserstoff oder einen Alkyl- oder Acylrest steht,

wobei die Alkyl-, Acyl- und Alkoxygruppen 1 bis 4 C-Atome, die Alkoxycarbonylgruppen 2 bis 3 C-Atome und die Cycloalkylgruppen 3 bis 6 C-Atome enthalten, mit der Ausnahme von

a) Verbindungen, in deren Formel gleichzeitig $R_1$ für Wasserstoff, Halogen oder einen Alkyl- oder Alkoxyrest,

$R'_2$ für den Methoxycarbonylmethyl- oder den Ethoxycarbonylmethylrest,

$R_3$ für Wasserstoff oder einen Alkylrest und

$R'_4$ für Wasserstoff steht, oder

b) Verbindungen, in deren Formel gleichzeitig $R_1$ für Wasserstoff, Halogen oder einen Alkyl- oder Alkoxyrest,

$R'_2$ für einen $CONHR_5$- oder $CH_2CONHR_5$-Rest, in dem $R_5$ Wasserstoff oder einen Alkyl-, Cycloalkyl- oder Benzylrest bedeutet,

$R_3$ für Wasserstoff oder einen Alkylrest und

$R'_4$ für Wasserstoff steht, oder

c) Verbindungen, in deren Formel gleichzeitig $R_1$ für Wasserstoff, Halogen oder einen Alkyl- oder Alkoxyrest,

$R'_2$ für den Ethoxycarbonylmethylrest,

$R_3$ für den Ethoxycarbonylrest und

$R'_4$ für Wasserstoff steht.

3. Pyrido-Indole nach Anspruch 2, in deren Formel

$R_1$ H, Cl, F, Br, $CH_3$, $CH_3O$ oder $CF_3$,

$R'_2$ $CH_2COOCH_3$, $CH_2COOC_2H_5$, $CONHCH_3$, $CH_2CONHCH_3$, $CONHC_2H_5$ oder $CH_2CONHC_2H_5$,

$R_3$ H, $CH_3$ oder $COOC_2H_5$ und

$R'_4$ H, $CH_3$ oder $CH_3CO$ bedeutet.

4. Verfahren zur Herstellung von Verbindungen nach Anspruch 2, bei welchen $R_3$ von H verschieden ist, dadurch gekennzeichnet, daß man eine Verbindung der Formel

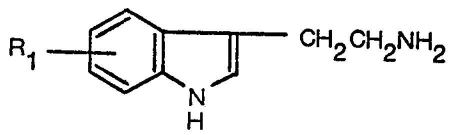

mit einer Verbindung der Formel $R_3CO(CH_2)_nCOOAlk$, in der n für 0 oder 1 und Alk für einen Alkylrest steht, umsetzt und anschließend gegebenenfalls die erhaltene Verbindung der Formel

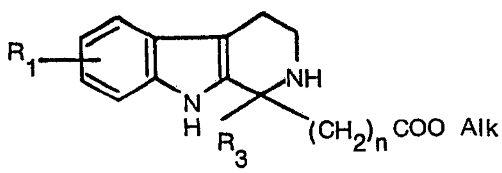

entweder mit einem Amin $R_5NH_2$ umsetzt, um eine Verbindung der formel (I bis) zu erhalten, in der $R'_2$ für $CONHR_5$ oder $CH_2CONHR_5$ steht, und/oder mit einer Verbindung $R'_4$ X (X=Anion) umsetzt, um eine Verbindung der Formel (I bis) zu erhalten, in der $R'_4$ für einen Alkyl- oder Acylrest steht, wobei die Symbole $R_1$, $R'_2$, $R_3$, $R'_4$ und $R_5$ die im Anspruch 2 genannte Bedeutung haben.

5. Verfahren zur Herstellung von Verbindungen nach Anspruch 2, bei welchen $R'_2$ für $CH_2COOAlkyl$ oder $CH_2CONHR_5$ und $R_3$ für Wasserstoff steht, dadurch gekennzeichnet, daß man eine Verbindung der Formel

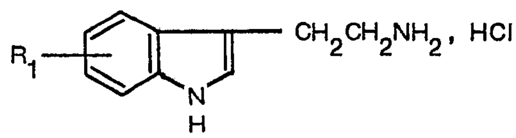

mit der Verbindung $C_2H_5O—CO—CO—CH_2—COOC_2H_5$ umsetzt, um eine Verbindung der Formel

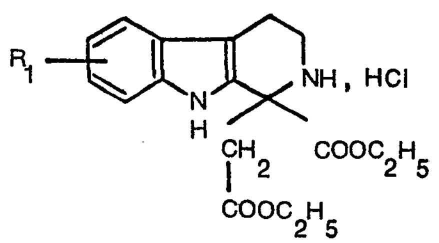

zu erhalten, die man zur Dicarbonsäure der Formel

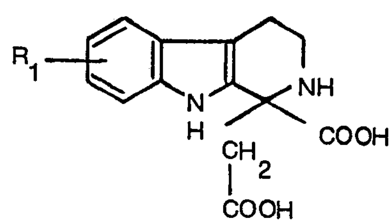

verseift, die ihrerseits mit einem Alkohol AlkOH umgesetzt wird, um eine Verbindung der Formel (I bis)

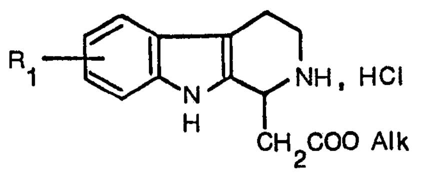

in der Alk für einen Alkylrest steht, zu erhalten, worauf diese Verbindung (I bis) gewünschtenfalls entweder mit einem Amin $R_5NH_2$ umgesetzt wird, um eine Verbindung der Formel (I bis) zu erhalten, in der $R'_2$ für $CH_2CONHR_5$ steht, und/oder mit einer Verbindung $R'_4$ X (X = Anion) umsetzt, um eine Verbindung der Formel (I bis) zu erhalten, in der $R'_4$ für einen Alkyl- oder Acylrest steht, wobei die Symbole $R_1$, $R'_4$ und $R_5$ die in Anspruch 2 angegebene Bedeutung haben.

**Patentansprüche für den Vertragsstaat: AT**

1. Verfahren zur Herstellung von Pyrido-Indolen in Form ihrer Racemate oder optisch aktiven Isomeren, die der allgemeinen Formel (I bis)

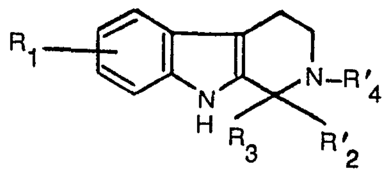

(I bis)

entsprechen, in welcher $R_1$ für Wasserstoff, Halogen, einen Alkyl-, Alkoxy- oder den Trifluormethylrest,

$R'_2$ für eine $CH_2COO$-Alkylgruppe oder die Reste $CONHR_5$ oder $CH_2CONHR_5$, in welchen $R_5$ Wasserstoff, einen Alkyl-, Cycloalkyl-, Benzyl-, Phenyl- oder Methoxyphenylrest bedeutet,

$R_3$ für einen Alkyl- oder Alkoxycarbonylrest und

$R'_4$ für Wasserstoff oder einen Alkyl- oder Acylrest steht,

wobei die Alkyl-, Acyl- und Alkoxygruppen 1 bis 4 C-Atome, die Alkoxycarbonylgruppen 2 bis 3 C-Atome und die Cycloalkylgruppe 3 bis 6 C-Atome enthalten, mit der Ausnahme von

a) Verbindungen, in deren Formel gleichzeitig $R_1$ für Wasserstoff, Halogen oder einen Alkyl- oder Alkoxyrest,

$R'_2$ für den Methoxycarbonylmethyl- oder den Ethoxycarbonylmethylrest,

$R_3$ für einen Alkylrest und

$R'_4$ für Wasserstoff steht, oder

b) Verbindungen, in deren Formel gleichzeitig $R_1$ für Wasserstoff, Halogen oder einen Alkyl- oder Alkoxyrest,

$R'_2$ für einen $CONHR_5$- oder $CH_2CONHR_5$-Rest, in dem $R_5$ Wasserstoff oder einen Alkyl-, Cycloalkyl- oder Benzylrest bedeutet,

$R_3$ für einen Alkylrest und

$R'_4$ für Wasserstoff steht, oder

c) Verbindungen, in deren Formel gleichzeitig $R_1$ für Wasserstoff, Halogen oder einen Alkyl- oder Alkoxyrest,

$R'_2$ für den Ethoxycarbonylmethylrest,

$R_3$ für den Ethoxycarbonylrest und

$R'_4$ für Wasserstoff steht, dadurch gekennzeichnet, daß man eine Verbindung der Formel

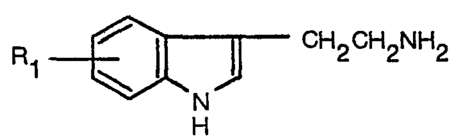

mit einer Verbindung der Formel $R_3CO(CH_2)_nCOOAlk$, in der n für 0 oder 1 und Alk für einen Alkylrest steht, umsetzt und anschließend gegebenenfalls die erhaltene Verbindung der Formel

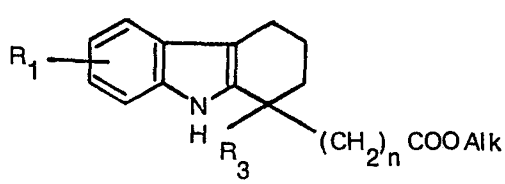

entweder mit einem Amin $R_5NH_2$ umsetzt, um eine Verbindung der Formel (I bis) zu erhalten, in der $R'_2$ für $CONHR_5$ oder $CH_2CONHR_5$ steht, und/oder mit einer Verbindung $R'_4$ X (X=Anion) umsetzt, um eine Verbindung der Formel (I bis) zu erhalten, in der $R'_4$ für einen Alkyl- oder Acylrest steht, wobei die Symbole $R_1$, $R'_2$, $R_3$, $R'_4$ und $R_5$ die oben genannte Bedeutung haben.

2. Verfahren zur Herstellung von Pyrido-Indolen in Form ihrer Racemate oder optisch aktiven Isomeren, die der allgemeinen Formel (I bis)

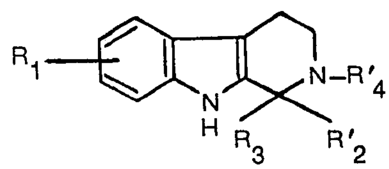

entsprechen, in welcher $R_1$ für Wasserstoff, Halogen, einen Alkyl- oder Alkoxy- oder den Trifluoromethylrest,

$R'_2$ für eine $CH_2COO$-Alkylgruppe oder den Rest $CH_2CONHR_5$, in welchem $R_5$ Wasserstoff, einen Alkyl-, Cycloalkyl-, Benzyl-, Phenyl- oder Methoxyphenylrest bedeutet,

$R_3$ für Wasserstoff und

$R'_4$ für Wasserstoff oder einen Alkyl- oder Acylrest steht, wobei die Alkyl-, Acyl- und Alkoxygruppen 1 bis 4 C-Atome, die Alkoxycarbonylgruppen 2 bis 3 C-Atome und die Cycloalkylgruppe 3 bis 6 C-Atome enthalten, mit der Ausnahme von

a) Verbindungen, in deren Formel gleichzeitig $R_1$ für Wasserstoff, Halogen oder einen Alkyl- oder Alkoxyrest,

$R'_2$ für den Methoxycarbonylmethyl- oder den Ethoxycarbonylmethylrest, und

$R'_4$ für Wasserstoff steht, oder

b) Verbindungen, in deren Formel gleichzeitig $R_1$ für Wasserstoff, Halogen oder einen Alkyl- oder Alkoxyrest,

$R'_2$ für einen $CONHR_5$- oder $CH_2CONHR_5$-Rest, in dem $R_5$ Wasserstoff oder einen Alkyl-, Cycloalkyl- oder Benzylrest bedeutet, und

$R'_4$ für Wasserstoff steht, dadurch gekennzeichnet, daß man eine Verbindung der Formel

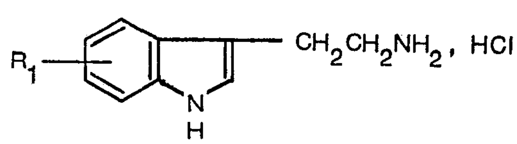

mit der Verbindung $C_2H_5O—CO—CO—CH_2—COOC_2H_5$ umsetzt, um eine Verbindung der Formel

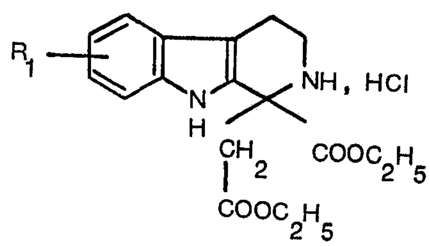

zu erhalten, die man zur Dicarbonsäure der Formel

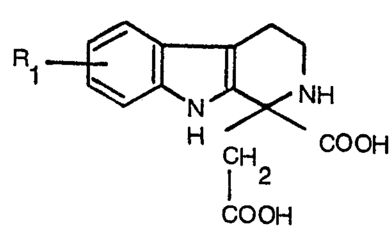

verseift, die ihrerseits mit einem Alkohol AlkOH umgesetzt wird, um eine Verbindung der Formel (I bis)

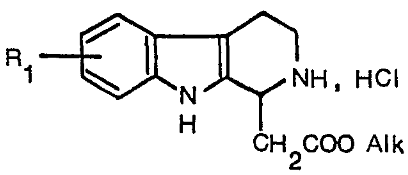

in der Alk für einen Alkylrest steht, zu erhalten, worauf diese Verbindung (I bis) gewünschtenfalls entweder mit einem Amin $R_5NH_2$ umgesetzt wird, um eine Verbindung der Formel (I bis) zu erhalten, in der $R'_2$ für $CH_2CONHR_5$ steht, und/oder mit einer Verbindung $R'_4$ X (X = Anion) umsetzt, um eine Verbindung der Formel (I bis) zu erhalten, in der $R'_4$ für einen Alkyl- oder Acylrest steht, wobei die Symbole $R_1$, $R'_4$ und $R_5$ die oben angegebene Bedeutung haben.